# EUROPEAN PATENT SPECIFICATION

(11) **EP 4 161 596 B1**
(45) Date of publication and mention of the grant of the patent: **06.08.2025**
(21) Application number: 21734269.0
(22) Date of filing: 25.05.2021
(51) Int. Cl.: A61L 15/26, A61L 15/46, A61K 9/00

(54) **NANOFIBROUS MATERIAL PARTICULARLY FOR TOPICAL USE IN THERAPIES**
NANOFASERMATERIAL, INSBESONDERE ZUR TOPISCHEN VERWENDUNG IN THERAPIEN
MATÉRIAU NANOFIBREUX DESTINÉ EN PARTICULIER À UNE UTILISATION TOPIQUE DANS DES THÉRAPIES

(30) Priority: 04.06.2020 CZ 20200031
(43) Date of publication of application: 12.04.2023
(73) Proprietor: Univerzita Karlova, 116 36 Praha 1 (CZ); Ústav organické chemie a biochemie Akademie ved Ceske Republiky, v.v.i., 16000 Prague (CZ); Technicka Univerzita v Liberci, 46117 Prague (CZ)
(72) Inventor: ZAJICEK, Robert, 18200 Prague (CZ); GAL, Peter, 04001 Kosice (SK); REJMAN, Dominik, 17000 Prague (CZ); DO PHAM, Duy Dinh, 13000 Prague (CZ); MOJR, Viktor, 33601 Letiny (CZ); HAVLICKOVA, Kristyna, 38232 Velesin (CZ); JENCOVA, Vera, 46343 Prosec pod Jestedem (CZ); LUKAS, David, 46006 Liberec (CZ); ASATIANI, Nikifor, 46015 Liberec (CZ); MIKES, Petr, 46331 Mnisek (CZ); KUZELOVA KOSTAKOVA, Eva, 51101 Turnov (CZ)
(74) Representative: Harber IP s.r.o.
(86) International application number: PCT/CZ2021/050055
(87) International publication number: WO 2021/244684

(56) References cited:
- WO-A1-2020/037218
- GÁMEZ ENRIQUE ET AL: "Antimicrobial Electrospun Polycaprolactone-Based Wound Dressings: An In Vitro Study About the Importance of the Direct Contact to Elicit Bactericidal Activity", ADVANCES IN WOUND CARE, vol. 8, no. 9, 1 September 2019 (2019-09-01), pages 438 - 451, XP055831650, ISSN: 2162-1918, Retrieved from the Internet <URL:https://www.ncbi.nlm.nih.gov/pmc/articles/PMC6855275/pdf/wound.2018.0893.pdf> DOI: 10.1089/wound.2018.0893
- ZBORNÍKOVÁ EVA ET AL: "Evaluation of Second-Generation Lipophosphonoxins as Antimicrobial Additives in Bone Cement", ACS OMEGA, vol. 5, no. 7, 25 February 2020 (2020-02-25), US, pages 3165 - 3171, XP055831651, ISSN: 2470-1343, Retrieved from the Internet <URL:https://pubs.acs.org/doi/pdf/10.1021/acsomega.9b03072> DOI: 10.1021/acsomega.9b03072

## Description

### Technical Field

The invention relates to nonwoven fabrics made of macromolecular materials and carriers of pharmaceutically active substances for topical use in therapies.

### Background Art

Nonwoven fabrics based on nanofibrous polymeric materials are widely used in healthcare. Because they are formed by a network of fibers with a diameter of tens to hundreds of nanometers, they are able to mechanically prevent the penetration of microorganisms such as bacteria and viruses. For this reason, they are used, for example, as protective membranes in the treatment and healing of wounds, dressings, filters, and substrates for tissue engineering. It is important that the material used to make the fabric meets the condition of biocompatibility. Therefore, polymers and copolymers based on lactic acid (PLA, PLLA, PDLLA) are most often used. However, the use of polyethylene glycol (PEG), polyurethane (PU), polyvinyl acetate (PVAc), polyvinyl alcohol (PVA), polyvinylpyrrolidone (PVP), polycaprolactone (PCL), acetate fibers (CA), hypromellose (HPMC), and gelatin have also been described.

The most commonly used method of preparing polymeric nanofibers is electrospinning. The method is based on using an electrode in the form of a capillary and a metal collector, while high voltage is applied between the two. Polymer solution is extruded from the capillary and formed into a thin stream of liquid by the electric field. Subsequently, the solvent evaporates and thin polymer fibers are formed and trapped on the collector.

Another possible use of polymeric nanofibers, specifically their use as carriers for pharmaceutically active substances, is currently being studied. The advantage is an easy handling of the drug in such a form, topical application of the drug, and in the case of more complex systems also a controlled release. Materials carrying, for example, heparins, analgesics, nonsteroidal antirheumatic drugs, and anticancer drugs are known. Furthermore, nanofibers carrying antibiotics such as β-lactam antibiotics, tetracycline antibiotics, or rifamycins have been studied.

The simplest methods for preparing nanofibers carrying pharmaceutically active substances include immobilizing the drug on pre-formed nanofiber fabrics or mixing the drug into a solution of a polymer used for electrospinning. The advantage of such procedures is the possibility of using basic instrumentation. The disadvantage is that the drugs are released from the resulting material by a simple diffusion. The release profile, which in this case is not externally controlled, is characterized by a very rapid increase in the concentration of drug in the surrounding solution, which can lead to a release of up to 70% of the incorporated drug during the first hour, as described for example in J. Zeng et al., INFLUENCE OF THE DRUG COMPATIBILITY WITH POLYMER SOLUTION ON THE RELEASE KINETICS OF ELECTROSPUN FIBER FORMULATION. This is followed by a rapid decrease in the release rate, which subsequently stabilizes at a significantly lower value. This can be a problem for drugs with a narrow therapeutic range, where the initial rapid increase in concentration can reach toxic values and subsequently fall below the therapeutic level. This problem can be solved by using controlled-release materials, which, for example, require the preparation of fibers covered with a diffusion-preventing layer, while the dosing is controlled by disintegration of the polymeric fibers. However, the preparation of such materials requires special procedures and more complex, and therefore more expensive, instrumentation.

Nanofibers carrying antibiotics can be used as covering membranes in the treatment and healing of wounds, as they prevent the growth of microorganisms in the wound and the development of infection. However, the use of materials that release antibiotics in an uncontrolled manner by diffusion carries the risk of developing bacterial resistance, as the drug concentration rapidly drops below the effective level due to the release profile. It is therefore necessary to change the carrier frequently. Likewise, a large amount of the substance released from the fresh carrier can cause complications. Last but not least, the use of these materials in therapies is problematic due to the low thermal and UV stability of some active substances, which prevents the possibility of their sterilization before the therapeutic use itself.
Gámez E. et al., ADVANCES IN WOUND CARE, VOLUME 8, NUMBER 9, discloses a nanofibrous material for topical use in skin wounds and ulcers, comprising electrospun PCL nanofibers loaded with 20 % (w/w) carvacrol or thymol as antimicrobacterial agents. WO 2020/037218 A1 discloses electrospun polyester nanofibers (such as PCL) with an active antibiotic agent, such as rifampin, linezolid, vancomycin or daptomycin.

Lipophosphonoxins (LPPO), described for example in documents EP2527351 and EP3448865, are a new group of antibacterial peptidomimetics. These substances have strong antibacterial effects against gram-positive and gram-negative bacteria, are not genotoxic, and their use is associated with a very low probability of developing a bacterial resistance. Furthermore, these substances are very stable to high temperatures and UV radiation. Synthetic preparation is undemanding and their modularity allows for tuning of their biological properties.
Zborníková et al., ACS Omega 2020, 5, 3165-3171, describes lipophosphonoxins as antimicrobial additives in bone cement in orthopedic implants.

The object of the invention is to provide a material for topical therapies of infectious diseases and treatment of skin burns that overcomes the above-mentioned drawbacks of the prior art.

### Summary of Invention

The present invention overcomes the shortcomings identified in the prior art by combining a polycaprolactone nanofibrous material with drugs belonging to the group of lipophosphonoxins (LPPO). The combination of these two components results in a material with non-obvious properties that differ fundamentally from other drugs incorporated into polymeric nanofibrous materials. By combining LPPO with any other polymeric material used to prepare prior-art nanofibers with incorporated drugs, the effect of the invention is not achieved.

The group of lipophosphonoxins includes substances of general formula I, 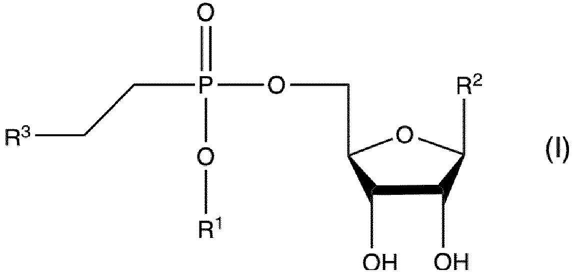
wherein R¹ is selected from a group comprising (C₈-C₂₂)alkyl, hexadecyloxypropyl, tetradecyloxypropyl, tetradecyloxyethyl, or hexadecyloxyethyl,
R² is selected from a group comprising uracil, thymine, cytosine, and
R³ is selected from a group comprising primary, secondary, tertiary amine, diastereomers and mixtures of diastereomers of compounds according to general formula I, and their pharmaceutically acceptable salts and hydrates.

To achieve the effect of the invention, substituent R¹ formed by a long hydrophobic hydrocarbon chain is the key component. Polycaprolactone also contains a hydrophobic hydrocarbon chain in its molecule, and therefore substances belonging to the LPPO group, the molecule of which contains both a hydrophilic and a hydrophobic part, bind very tightly to polycaprolactone chains by the hydrophobic part of the molecule due to non-covalent interactions. The result is a material that does not release the drug by a simple diffusion mechanism. It was experimentally confirmed that even after five days, no detectable amount of LPPO was released into the solution in aqueous medium. In this case, LPPO can only be released by degradation of the polymeric material, specifically by its hydrolytic decomposition. Polycaprolactone is relatively resistant to spontaneous hydrolysis in an aqueous environment and measurable decomposition occurs in months or years. In contrast, it is prone to enzymatic hydrolysis, for example by lipases. Thus, in an aqueous medium containing lipases, the polymer is degraded and the incorporated drug is released, the rate of which is directly proportional to the enzymatic activity in solution.

When using LPPO-carrying polycaprolactone-based materials in the topical treatment of infectious skin diseases, an effect occurs which is not observed in therapeutic drug-carrying polymer nanofibers known from the prior art. At the site of infection, the activity of lipases and other hydrolytic enzymes is increased due to the increased immune response of the organism. Thus, LPPO is released at a rate proportional to the intensity of the immune response at the site of action. Conversely, after the infection subsides and the inflammatory immune response of the organism is attenuated at the site of action, the release of the incorporated LPPO from the material is slowed down and eventually halted.

Experiments monitoring the weight loss of a polycaprolactone material (PCL), carrying LPPO by comparison with control samples of pure PCL and PCL carrying L-arginine have shown that there is no measurable release of LPPO into solution in an aqueous medium. In contrast, the L-arginine-carrying sample experienced significant weight loss, because L-arginine does not form strong bonds with the polyester chains, and was therefore prone to release by a simple diffusion mechanism. Furthermore, it was shown that in the aqueous medium in the presence of lipase, there is a significant weight loss of the tested material, while the sample containing LPPO showed the highest rate of degradation of all three samples. Thus, the properties of LPPO contribute to an increase in the degradative effect of hydrolytic enzymes on polyester fibers, which contributes to easier drug release during therapies.

Suitable forms of therapeutic preparations based on polyester fibers carrying LPPO are nanofiber nonwoven fabrics formed by fibers preferably with a diameter of less than 5 µm. These can serve as an active covering material to prevent the development of early infection, for example in postoperative wounds, invasive entrances, or after skin transplantation. Furthermore, they can be used in the treatment of traumatic skin loss, for example burns, or in the treatment of acute and chronic skin diseases, for example ischemic skin defects, skin ulcers, or diabetic foot.

The above-mentioned fabrics also find use as a secondary coating for artificial skin prostheses, since they prevent the development of infection, while none of the components, i.e. polycaprolactone and LPPO, are toxic to skin cells and they do not prevent multiplication of skin cells.

Another suitable formulation for topical therapies are gels containing PCL nanofibers carrying LPPO, which can be useful, for example, in ophthalmology in the treatment of infected corneal defects.

The combination of PCL and LPPO for the production of coating materials for therapeutic use is also beneficial for another reason. Both components have high stability to high temperatures and UV radiation, and thus allow easy sterilization before use in therapy.

The production of polycaprolactone nanofibers with incorporated LPPO is possible using a simple method of electrospinning from a mixture containing a solution of the desired polymer and LPPO. Thus, it is not necessary to use more complicated, and therefore more expensive, manufacturing methods, which are necessary in the prior art to produce controlled-release fibers.

### Brief Description of Drawings

**Fig.1**

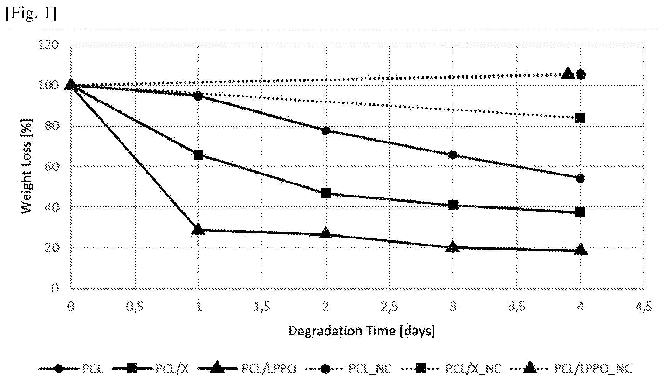

   [Fig.1] depicts a graph of weight loss of samples of polycaprolactone-based (PCL) nanofibrous polymeric material according to Examples 2 and 3. PCL_NC represents a sample of pure PCL incubated in phosphate buffer; PCL/X_NC represents a sample of PCL carrying incorporated L-arginine in phosphate buffer; PCL/LPPO_NC represents a sample of PCL carrying incorporated LPPO in phosphate buffer; PCL represents a sample of pure PCL incubated in phosphate buffer containing lipase enzyme; PCL/X represents a sample of PCL carrying incorporated L-arginine in a phosphate buffer containing lipase enzyme; PCL/LPPO represents a sample of PCL carrying incorporated LPPO in phosphate buffer containing lipase enzyme.
**Fig.2**

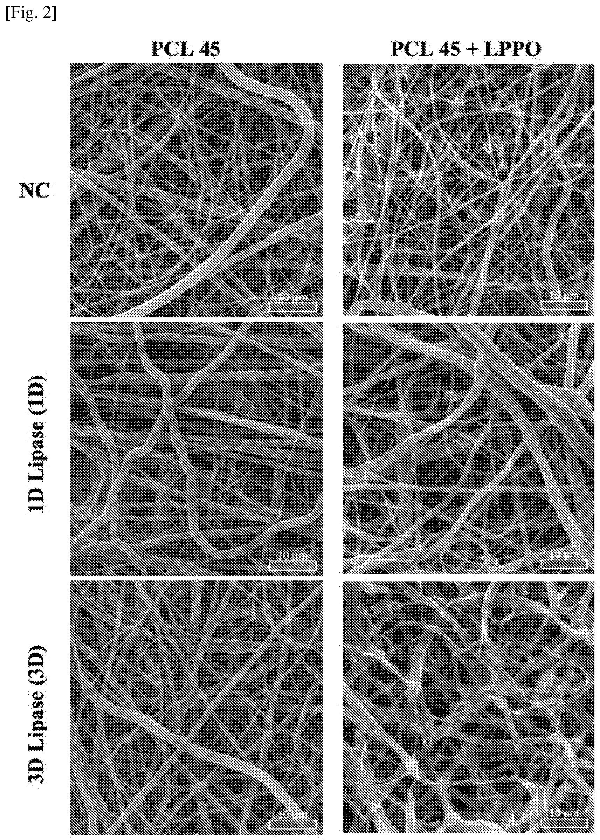

   [Fig.2] depicts electron microscope images of samples of pure PCL-based material and PCL-based material carrying LPPO after incubation in phosphate buffer and in phosphate buffer containing lipase enzyme according to Examples 2 and 3.
**Fig.3**

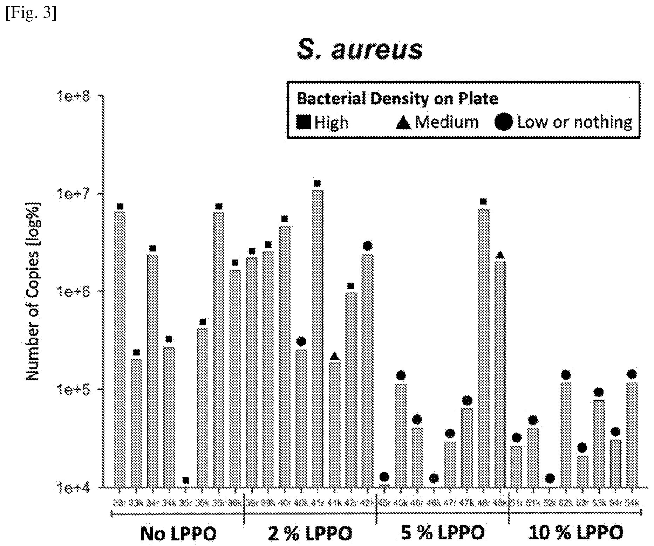

   [Fig.3] depicts qPCR values and results of bacteriological analysis of skin wounds of mice inoculated with S. aureus after 7 days of therapy using a PCL-based material carrying LPPO at various concentrations according to Example 4.

### Description of Embodiments

### Example 1

Example 1 describes a process of preparation of nanofibrous polymeric material based on polycaprolactone (PCL) carrying LPPO.

Selected LPPO is dispersed in a solvent system of chloroform: methanol 8:2. The amount is chosen so that the LPPO constitutes 2, 5, or 10wt.%, respectively, in the dry matter relative to the polymer. Next, polycaprolactone with an average molecular weight of 45 kDa and a concentration of 16wt.% relative to the solvent is added to the suspension. The material is spun on an electrospinning machine at a potential difference of 40 kV between electrode and collector with a positive charge applied to the electrode and a negative charge to the collector. The distance between the electrode and the collector is 160 mm. The produced samples are then sterilized by UV light in a flow box for 10 minutes on each side.

### Example 2

Example 2 describes an experiment monitoring the release of LPPO from a PCL-based polymeric material by simple diffusion.

Following the procedure described in Example 1, three samples are prepared for testing - pure PCL-based nanofibrous material, PCL-based nanofibrous material carrying L-arginine (22wt.%), and PCL-based nanofibrous material carrying LPPO (11wt.%). A50 mg sample of each material is placed in a 15 mL tube and 5 mL of phosphate buffer (PBS, pH 7.4) is added. The samples are then incubated at 37 °C for 5 days, while the incubation medium is changed to a fresh one every 24 h and the collected medium is analyzed by HPLC. At the end of the incubation period, the samples are removed, rinsed with distilled water, and dried to a constant weight. For the dried samples, mass analysis and morphology analysis using an electron microscope are performed.

### Example 3

Example 3 describes an experiment monitoring enzymatic degradation of a PCL-based polymeric material carrying LPPO.

Following the procedure described in Example 1, three samples are prepared for testing - pure PCL-based nanofibrous material, PCL-based nanofibrous material carrying L-arginine (22wt.%), and PCL-based nanofibrous material carrying LPPO (11wt.%). A 50 mg sample of each material is placed in a 15 mL tube and 5 mL of phosphate buffer (PBS, pH 7.4) containing lipase enzyme from Pseudomonas cepacia (30 U/mL) is added. The samples are then incubated at 37 °C for 5 days, while the incubation medium is changed to a fresh one every 24 h and the collected medium is analyzed by HPLC. At the end of the incubation period, the samples are removed, rinsed with distilled water, and dried to a constant weight. For the dried samples, mass analysis and morphology analysis using an electron microscope are performed.

### Example 4

Example 4 describes an experiment investigating the effect of a PCL-based polymeric material carrying LPPO on a skin injury inoculated with S. aureus in laboratory mice.

A group of 24 laboratory mice with skin injuries inoculated with S. aureus (2.6E+4 c.f.u./mL) is divided into four groups of six mice. In group No. 1, the wound is covered with PCL-based material without incorporated drug substance and further with a polyurethane-based protective film. In group No. 2, the wound is covered with PCL-based material containing 2wt.% of LPPO and further with a polyurethane-based protective film. In group No. 3, the wound is covered with a PCL-based material containing 5wt.% of LPPO and further with a polyurethane-based protective film. In group No. 4, the wound is covered with a PCL-based material containing 10wt.% of LPPO and further with a polyurethane-based protective film. After 7 days, the condition of the infected wound and the surrounding skin is analyzed both visually and by qPCR analysis detecting a presence of bacterial DNA.

After 7 days, groups No. 1 and No. 2 develop a severe infection; in group No. 3, the wound heals in four mice and shows no infection or secretion, in two mice the infection develops; in group No. 4, the wound heals in five mice and does not show any presence of infection or secretion, in one mouse the infection develops.

### Industrial Applicability

Polycaprolactone nanofibers with incorporated lipophosphonoxin antibiotics are useful as a material for a production of nonwoven fabrics and gels for topical treatment of infectious skin diseases and treatment of skin burns.

## Claims

1. Nanofibrous material particularly for topical use in therapies, **characterized in that** it consists of a compound selected from a group comprising
lipophosphonoxins of general formula I, 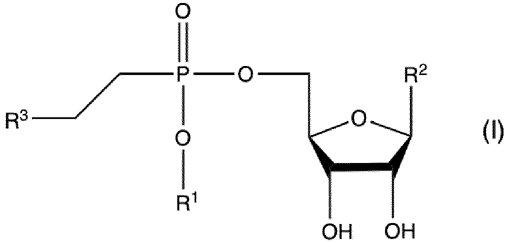
wherein R¹ is selected from a group comprising (C₈-C₂₂)alkyl,
hexadecyloxypropyl, tetradecyloxypropyl, tetradecyloxyethyl, hexadecyloxyethyl,
R² is selected from a group comprising uracil, thymine, cytosine, and
R³ is selected from a group comprising primary, secondary, tertiary amine,
diastereomers and mixtures of diastereomers of compounds according to general formula I, and their pharmaceutically acceptable salts and hydrates,
incorporated into nanofibers of polycaprolactone,.

2. Nanofibrous material according to claim 1, **characterized in that** it contains at least 5 wt. % of the lipophosphonoxin.

3. Nanofibrous material according to any of claims 1 or 2 for use in treatment of skin loss.

4. Nanofibrous material according to any of claims 1 or 2 for use in treatment of skin diseases.

5. Nanofibrous material according to any of claims 1 or 2 for use in application of artificial skin prostheses.

6. Nanofibrous material according to any of claims 1 or 2 for use in treatment of infected corneal defects.

## Patentansprüche

1. Nanofaseriges Material insbesondere zur topischen Anwendung in Therapien, **dadurch gekennzeichnet, daß** es aus einer Verbindung aus der Gruppe der Lipophosphonoxine der allgemeinen Formel I besteht, 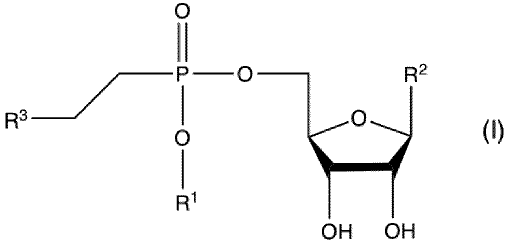
worin R¹ aus einer Gruppe ausgewählt ist, die (C₈-C₂₂)-Alkyl, Hexadecyloxypropyl, Tetradecyloxypropyl, Tetradecyloxyethyl, Hexadecyloxyethyl umfasst,
R² aus einer Gruppe ausgewählt ist, die Uracil, Thymin, Cytosin umfasst, und
R³ ausgewählt ist aus einer Gruppe, die primäres, sekundäres, tertiäres Amin umfasst,
Diastereomere und Mischungen von Diastereomeren von Verbindungen gemäß der allgemeinen Formel I, und
ihre pharmazeutisch akzeptablen Salze und Hydrate,
eingearbeitet in Polycaprolacton.

2. Nanofasermaterial nach Patentanspruch 1, **dadurch gekennzeichnet, dass** es mindestens 5 Gew.-% des Lipophosphonoxins enthält.

3. Nanofasermaterial nach einem der Patentansprüche 1 oder 2 zur Verwendung bei der Behandlung von Hautverlust.

4. Nanofasermaterial nach einem der Patentansprüche 1 oder 2 zur Verwendung bei der Behandlung von Hautkrankheiten.

5. Nanofasermaterial nach einem der Patentansprüche 1 oder 2 zur Verwendung bei der Herstellung von künstlichen Hautprothesen.

6. Nanofaseriges Material nach einem der Patentansprüche 1 oder 2 zur Verwendung bei der Behandlung von infizierten Hornhautdefekten.

## Revendications

1. Un matériau nanofibreux particulièrement destiné à une utilisation topique dans les thérapies, **caractérisé par le fait qu'**il est constitué d'un composé appartenant à un groupe comprenant des lipophosphonoxines de formule Générale I, 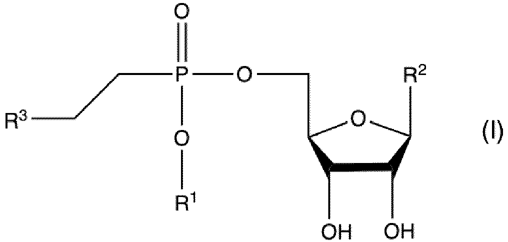
dans lequel R¹ est choisi dans un groupe comprenant (C₈-C₂₂)alkyle, hexadécyloxypropyle, tétradécyloxypropyle, tétradécyloxyéthyle, hexadécyloxyéthyle,
R² est choisi dans un groupe comprenant l'uracile, la thymine, la cytosine, et
R³ est choisi dans un groupe comprenant les amines primaires, secondaires et tertiaires,
les diastéréomères et les mélanges de diastéréomères des composés selon la formule générale I, et
leurs sels et hydrates pharmaceutiquement acceptables,
incorporés dans le polycaprolactone.

2. Le matériau nanofibreux selon la revendication 1, **caractérisé par le fait qu'**il contient au moins 5 % en poids de lipophosphonoxine.

3. Le matériau nanofibreux selon l'une des revendications 1 ou 2 pour utilisation dans le traitement de la perte de peau.

4. Le matériau nanofibreux selon l'une des revendications 1 ou 2 pour utilisation dans le traitement des maladies cutanées.

5. Le matériau nanofibreux selon l'une des revendications 1 ou 2 pour utilisation dans l'application de prothèses de peau artificielle.

6. Le matériau nanofibreux selon l'une des revendications 1 ou 2 pour utilisation dans le traitement des défauts cornéens infectés.
